Europäisches Patentamt

European Patent Office

Office européen des brevets

(11) Publication number: **0 072 498**
**B1**

# EUROPEAN PATENT SPECIFICATION

(45) Date of publication of patent specification: **20.04.88**

(51) Int. Cl.⁴: **G 01 S 7/52,** G 01 S 15/89,
G 10 K 11/34, G 10 K 11/32

(21) Application number: **82107057.0**

(22) Date of filing: **04.08.82**

(54) Ultrasonic imaging apparatus.

(30) Priority: **18.08.81 JP 129529/81**

(43) Date of publication of application:
**23.02.83 Bulletin 83/08**

(45) Publication of the grant of the patent:
**20.04.88 Bulletin 88/16**

(84) Designated Contracting States:
**DE FR NL**

(56) References cited:
**US-A-3 833 825**
**US-A-3 936 791**
**US-A-4 016 750**
**US-A-4 016 751**

**PATENTS ABSTRACTS OF JAPAN, vol. 5, no.**
**121(E-68)(793), 5th August 1981**

(73) Proprietor: **KABUSHIKI KAISHA TOSHIBA**
**72, Horikawa-cho Saiwai-ku**
**Kawasaki-shi Kanagawa-ken 210 (JP)**

(72) Inventor: **Sasaki, Hiroshi**
**3326-20, Mihara-cho**
**Ootawara-shi Tochigi-ken (JP)**

(74) Representative: **Henkel, Feiler, Hänzel & Partner**
**Möhlstrasse 37**
**D-8000 München 80 (DE)**

Courier Press, Leamington Spa, England.

## Description

The present invention relates to an ultrasonic imaging apparatus comprising: transducer means, including a linear array of transducer elements sequentially disposed along an axis for directing pulses of ultrasonic energy in the form of a beam at a plurality of ultrasonic frequencies into an object and receiving echoes of said pulses to convert said echoes to electrical signals, means for energizing said transducer means, filter means connected to said transducer means for filtering received electrical signals from said transducer means, signal processing means connected to said filter means for processing the frequency components of said electrical signals passed through said filter means to convert them into tomogram signals, and display means connected to said signal processing means for displaying a tomogram according to a tomogram signal from said signal processing means, said filter means selectively filtering said electrical signals and passing frequency components of said electrical signals associated with selected frequencies of said ultrasonic energy and not passing other frequency components of said electrical signals, the arrangement of said transducer elements and said filter means cooperating to cause the effective aperture of said transducer means for received echoes to vary with the selected frequency components of said electrical signals passed by said filter means.

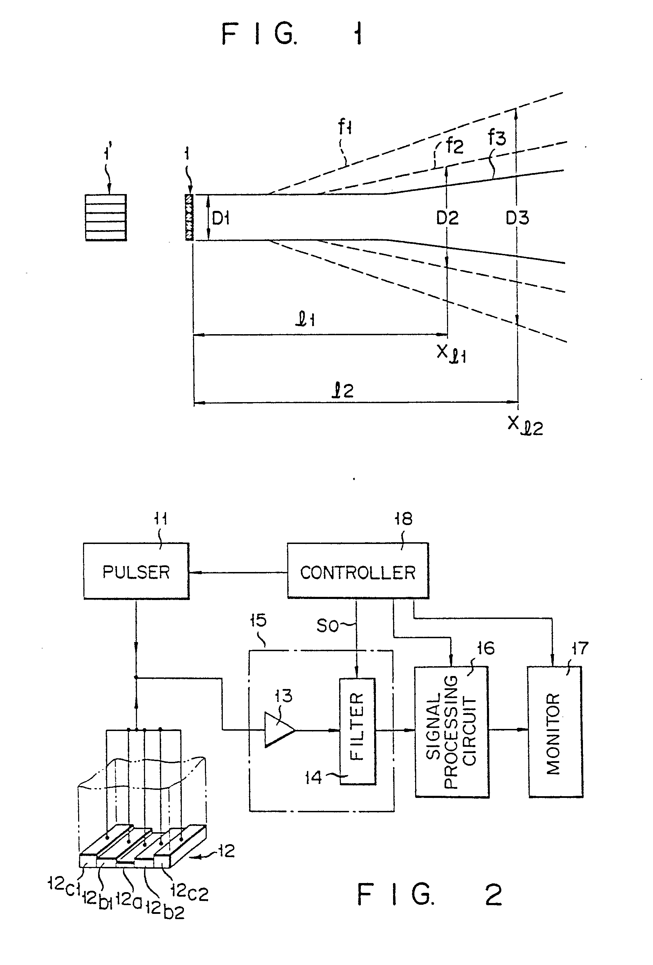

In an ultrasonic imaging apparatus, drive pulses generated by a pulse generator are applied to a transducer array, which is excited by these drive pulses to radiate ultrasound pulses into an object under diagnosis. The echo acoustic pulses returned from the object are received by the transducer array and converted into corresponding electrical signals. The converted electrical signals are properly processed to be visualized by a monitor in the form of a tomogram of the object. In this ultrasound imaging apparatus, a diameter of the ultrasonic beam radiated from the transducer array at a portion under diagnosis of the object influences the resolution of the tomogram in the azimuth direction, i.e. in a direction orthogonal to the propagating direction of the ultrasound beam, in such a way that the resolution of the tomogram is higher as the ultrasound beam converges at the portion under diagnosis. The diameter of the ultrasound beam is substantially equal to the width of the drive portion of the transducer in the vicinity of the transducer array. When the transducer is made up of a number of transducer elements, all of the driven transducer elements corresponds to the drive portion. In the one piece type transducer, the drive portion corresponds to the transducer portion. The resolution of the tomogram in the vicinity of the transducer array can be improved by making small a width of the drive portion of the transducer array. In the former type of the transducer, the resolution of the tomogram can be improved by reducing the number of the transducer elements. As the ultrasound beam emitted from the ultrasound transducer moves away from the transducer array, the diameter of the beam expands as it progresses. The expansion of the beam diameter is inversely proportional to the drive portion of the transducer array. Therefore, if the width of the drive portion is made small for improving the resolution at a location near the transducer, the expansion of the ultrasound beam diameter is large conversely and the resolution at a long range is deteriorated. Thus, for improving the resolution, contradiction is encountered that for improving the resolution in a short range, the width of the drive portion must be small, but for improving the resolution in a long range, it must be large. Further, the expansion of the ultrasound wave is inversely proportional to its frequency. Accordingly, the resolution in the azimuth direction also depends largely on the frequency of the ultrasound. This will be described in detail referring to Figure 1 illustrating an expansion (ultrasound field) of ultrasound beam radiated from a transducer array 1. The transducer array 1 is comprised of a plurality of transducer elements aligned in a series and with the same thickness, as viewed in the azimuth direction. A plan view of these arrayed transducer elements is indicated by reference numeral 1'. As seen from the figure, the diameter of the beam in the vicinity of the transducer array 1 is substantially equal to the width of the transducer array 1, irrespective of the frequency of the ultrasound wave. In a distant region from the transducer array, the beam changes its diameter with the frequency. In the figure, frequencies f1 to f3 of the ultrasound beams are larger with their suffix numbers, $f1 < f2 < f3$. As seem from the figure, the lower the frequency of the ultrasound beam, the larger the expansion of the beam. Therefore, to make the expansion of the beam diameter small, it is necessary to select the frequency of the beam to be high. In this case, however, another problem arises in that the attenuation of the ultrasound wave in a living body is larger with its frequency. This implies that the high frequency components of the ultrasound beam reduce at the measuring portion as it goes deeper in the living body. Accordingly, in a deep measuring portion of the living body, the low frequency components have great contribution to the resolution. In this respect, the frequency of the ultrasound beam must be limited below a given frequency. The consequence is a compromise of a proper beam diameter and a proper beam frequency. More explicitly, at a measuring portion $X_{l1}$ distanced "l1" from the transducer 1, the frequency component f3 is attenuated to almost zero. Accordingly, the resolution at this portion is determined by the frequency component at f2 with the beam diameter D2. Accordingly, the resolution at this measuring location depends on a compromise between the frequency f2 and the beam diameter D2. Further, at a measuring location $X_{l2}$ distanced "l2" from the transducer array 1, the frequency components f2 and f3 are both lost and only the frequency component f1 is effective. The resolution at the measuring location $X_{l2}$ is determined by the frequency component f1 and its diameter D3.

For the above background reason, the improvement of the resolution of a tomogram has a limit in both short and long ranges. Particularly, in the long range or in a deep measuring portion of the living body, a high resolution cannot be obtained and a picture quality of a tomogram is poor.

The earlier application corresponding to EP—A1—0 066 876 discloses an ultrasonic imaging apparatus similar to the apparatus as defined in the above opening passage. In other words, this earlier apparatus comprises a linear array of transducer elements sequentially disposed along an axis for directing pulses of ultrasonic energy in the form of a beam at a plurality of ultrasonic frequencies into an object and receiving echoes of said pulses to convert said echoes to electrical signals. The thickness of each of said transducer elements, which are energized by pulser means, increases stepwise with the distance from the center of the array. Each transducer element is connected through a series connection of a preamplifier and a delay circuit respectively to adding means. This adding means adds the output signals of the delay circuit, and supplies an input signal to display means through a series connection of filter means and signal processing means.

Document US—A—3,936,791 discloses a linear array of ultrasonic transducer elements in which the ultrasonic beam is focussed in elevation by a cylindrical lens and the resolution is thereby improved. Instead of a lens, a curved surface transducer may be used. There is however no suggestion that the elements have the broad band properties necessary for carrying out the present invention.

Documents US—A—4,241,610 and US—A—4,305,296 disclose ultrasonic imaging systems in which the ultrasonic wave transmission outlet diameter is varied in response to the depth of the region being scanned. The reason for this is that as the outlet diameter increases, the scanning wave can be transmitted deeper without diffusion. Thus, the deeper the region, the larger the outlet diameter is made, and the shallower this region, the smaller the outlet diameter is made. In the ultrasonic imaging systems shown in these two US-documents, a plurality of transducer elements is arranged along an axis which is parallel to the scanning plane. The transducer elements are mutually isolated so that it is possible to vary the size of the transmission outlet in a parallel direction by choosing which transducer elements are excited.

Furthermore, document US—A—4,016,751 describes an apparatus for ultrasonic examination of objects, particularly in medical diagnostic examination. This apparatus includes a 2-focus-transducer composed of several transducer parts, in order to reduce the widths of the beam over an extended distance.

It is an object of the present invention to provide an ultrasonic imaging apparatus which has an improved resolution in the azimuth direction.

According to the present invention, there is provided an ultrasonic imaging apparatus as defined above in the opening passage and which is characterized in that the thickness of each of said transducer elements varies continuously in a direction orthogonal to said array direction and to said beam so as to increase from a central portion thereof toward both edges.

The invention can be more fully understood from the following description when taken in conjunction with the accompanying drawings, in which:

Figure 1 shows an explanatory diagram of an ultrasound field formed by a prior transducer array;

Figure 2 is a block diagram illustrative of the principles of the invention and showing an ultrasonic imaging apparatus;

Figure 3 shows an explanatory diagram of an ultrasonic imaging apparatus shown in Figure 2;

Figure 4 shows a graph illustrating frequency distributions of ultrasounds radiated from a transducer;    •

Figure 5 shows a graph illustrating a filter characteristic of a filter used in the imaging apparatus;

Figure 6 shows a circuit diagram of a filter;

Figure 7 shows a filter characteristic of the Figure 6 filter;

Figures 8A to 8D show transducer elements for use in imaging apparatus according to the invention; and

Figure 9 shows a perspective view of a transducer using a plurality of the transducer elements shown in Figures 8A to 8D.

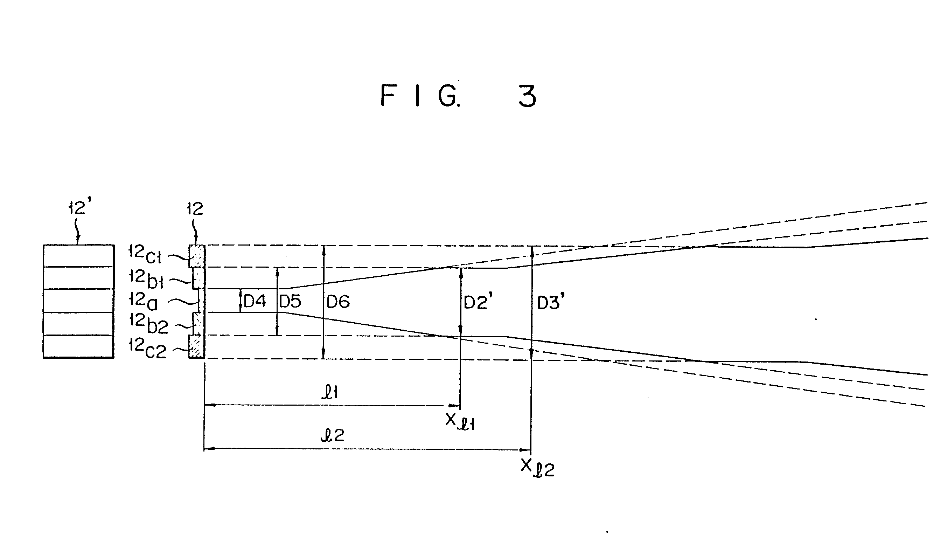

Referring to Figure 2, a pulser 11 produces a drive pulse at a fixed frequency rate, and is connected at the output terminal to a transducer 12 and to the input terminal of an amplifier 13. The output terminal of the amplifier 13 is connected to the input terminal of a band pass filter 14. The amplifier 13 and the filter 14 make up a receiver 15. The output terminal of the band pass filter 14 is connected to the input terminal of a signal processing circuit 16. The processing circuit 16 is connected to the input terminal of a monitor i.e. a display device 17. A controller 18 controls the operations of the pulser 11, the band pass filter 14, the signal processing circuit 16 and the monitor 17.

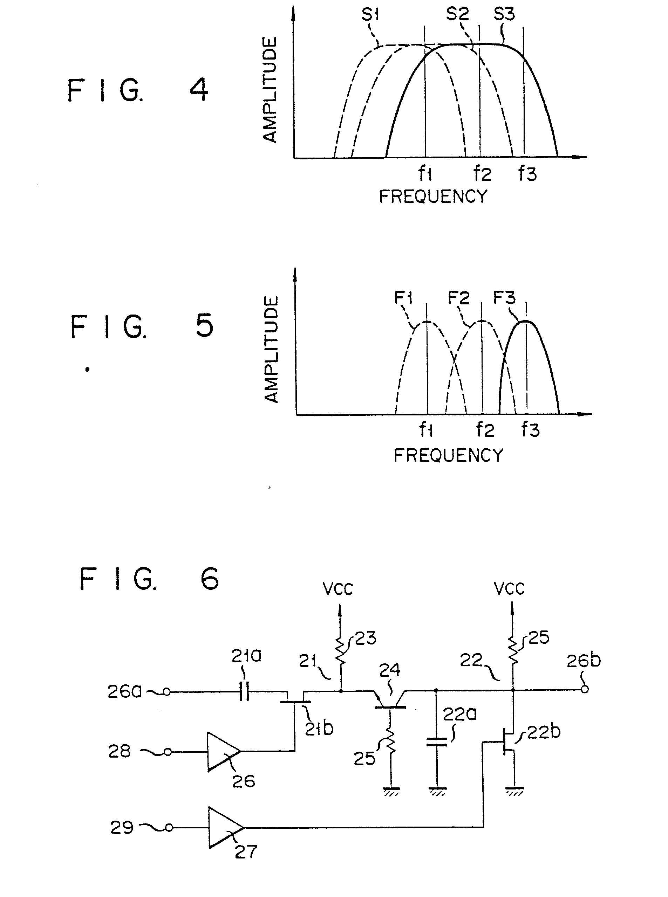

As shown, the transducer 12 has a plurality of transducer elements 12a, 12b1, 12b2, 12c1 and 12c2, and these elements are arrayed such that the thickness of these elements is thinner as the elements are located innerly, and the element 12a located at the center of the transducer 12 is the thinnest. The transducer 12 may be either of the separate type as illustrated or of the one-piece type. The filter 14 is of the dynamic type in which its center frequency shifts under control of the controller 18, with the same frequency band. As shown in Figure 5, the center frequency shifts from f1 to f3 with its associated band pass characteristic curves F1 to F3.

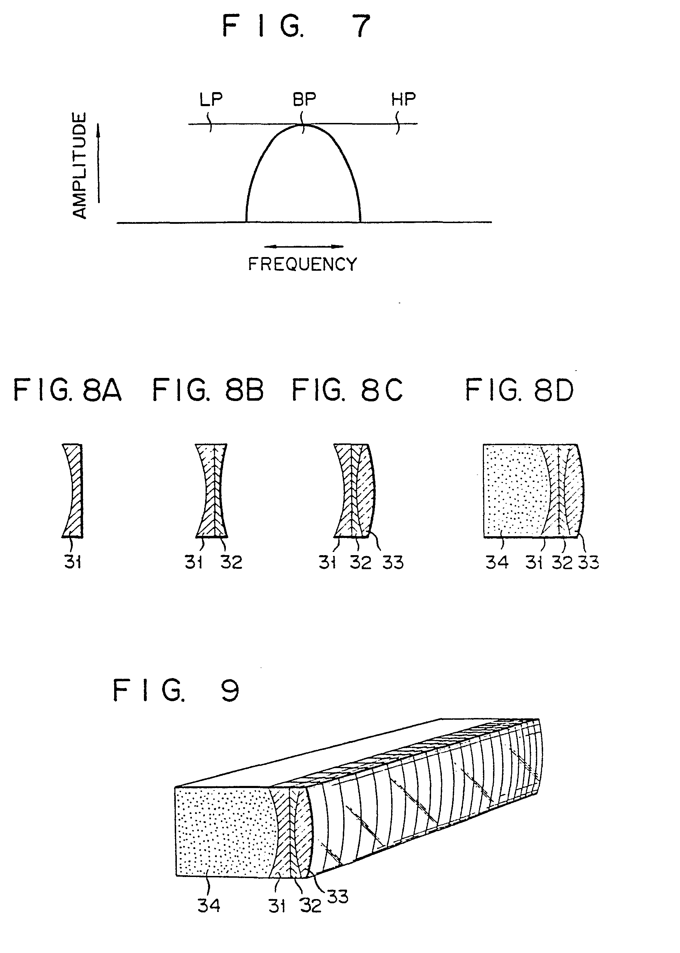

The band pass filter 14 is comprised of a high pass filter 21 and a low pass filter 22, as shown in, for example, Figure 6. Reference numerals 26a and 26b designate input and output terminals of the filter 14, respectively. The high pass filter 21 is comprised of serially connected capacitor 21a and impedance element, e.g., FET 21b. The output terminal of FET 21b is connected to a power source $V_{CC}$ through a resistor 23 and also to the emitter of a transistor 24, the base of which is grounded through a resistor 25. The collector of the transistor 24 is grounded passing in parallel through a capacitor 22a and FET 22b of the low pass filter 22, and is also connected to an ouput terminal 26b. The collector of the transistor 24 is connected to a power source $V_{CC}$ through a resistor 25. Control signal input terminals 28 and 29 are connected to the gates of FETs 21b and 22b through amplifiers 26 and 27, respectively. When predetermined variable signals, for example saw tooth signals, are supplied from the controller 18 to the control signal input terminals 28 and 29, the impedances of the FETs 21b and 22b vary, so that the filtering characteristics of the high pass filter 21 and low pass filter 22 vary. As a result, as shown in Figure 7, a band pass region BP, i.e. an overlap region of a high pass region HP and a low pass region LP, is shifted. Thus, a signal corresponding to the band pass region BP is obtained from the output terminal 26b.

The operation of the ultrasound imaging apparatus as shown in Figure 2 will be described.

The pulser 11 produces drive pulses at a fixed rate under control of the controller 18. The drive pulse from the pulser 11 is applied to the transducer elements 12a, 12b1, 12b2, 12c1 and 12c2. Upon receipt of the drive pulse, these elements produces ultrasound waves at respective frequencies. The ultrasound waves radiated propagate through a living body and return from many portions in the living body with different acoustic impedance in the form of echoes. The echoes are received by the transducer array 12 where these are converted into corresponding electrical signals. The converted signals are inputted into the amplifier 13 and then applied to the band pass filter 14. The band pass filter 14 filters the signals from the amplifier to pass only the signal components at a frequency within a given frequency band. The filtered out signals are properly processed and then applied to the monitor 17 where these are displayed.

An ultrasound field formed by the ultrasound transducer shown in Figure 2 is as shown in Figure 3. Incidentally, a plan view of the transducer 12 is indicated by reference numeral 12'. The frequency spectrum of the ultrasound wave radiated by the transducer elements is distributed such that as the transducer elements are thinner, the spectrum of the ultrasound waves radiated from the transducer elements has higher frequency components. This is well illustrated in Figure 4. In the figure, a curve denoted as S1 shows that the signal radiated in Figure 3 from the thickest transducer elements 12c1 and 12c2 located at the outer side of the transducer array 12 is in the lower frequency region. A curve denoted as S2 corresponds to the ultrasound radiated from the elements 12b1 and 12b2 and is located in a higher frequency region than the curve S1. A curve S3 is for the ultrasound radiation from the thinnest element 12a and is located at the highest frequency region. These transducer elements are concurrently driven to form a resultant curve of these curves S1 to S3. In Figure 4, f1 to f3 indicates the center frequency of the frequency bands of the filter 14 of which characteristics are illustrated in Figure 5. Individual positions in the ultrasound field have different spectra. If the filter characteristic of the filter 14 is set to the curve F3 with the center frequency f3 shown in Figure 5, the ultrasound component radiated from the transducer element 12a is received and the effective width of the transducer array 12 is D4 as shown in Figure 3 which is the diameter of the ultrasound beam from the thinnest element 12a. If the curve F2 with the center frequency f2 is selected, the effective width of the transducer 12 is D5 which is the sum of the diameters of the beams from the transducer elements 12a and 12b1 and 12b2. Similarly, the filter 14 is set to have the characteristic curve F3 with the center frequency f3, the effective width of the transducer is D6 the sum of the widths of the beams from the transducer elements 12a, 12b1, .12b2, 12c1 and 12c2. With this combination of the transducer elements with different widths and the filter 14 with a variable frequency band, the effective width of the transducer can be adjusted by changing the center frequency of the filter 14.

In this way, for taking a tomogram of a portion of the living body in the vicinity of the transducer 12, the filter 14 is set to the center frequency f3. At this time, the portion can be tomographed with the transducer width of D4 equal to the diameter of the beam from the thinnest transducer element 12a. The resolution of the tomogram taken is defined by the diameter D6. For tomographing a portion located at a position $X_{l1}$ distanced l1 from the transducer 12, the center frequency f2 is selected for the filter 14. The diameter D5 defines the resolution of tomogram taken. Similarly, for taking a tomogram of a portion at a position $X_{l2}$ distanced l2 from the transducer 12, the center frequency f3 is selected for the filter 14: A tomogram taken has a resolution depending on the diameter D6. In this manner, the center frequency of the frequency band of the filter 14 is selected according to a depth of a desired portion to be diagnosed from the transducer 12, thereby to have the best resolution at that portion.

The band pass filter 14 in the above-mentioned embodiment may be replaced by a high pass filter of which the cut off frequency is variable.

In one embodiment of the invention a transducer element 31 as shown in Figure 8A has a cross section continuously and inwardly curved at one side. A number of the transducer elements 31 with such a configuration are arrayed as shown in Figure 9 when used as a transducer. It will be noted that the thickness of each transducer element (31) varies continuously in a direction orthogonal to said array direction and to said beam so as to increase from the central portion towards both edges. A transducer element shown in Figure 8B is a modification of the transducer element shown in Figure 8A, and uses additionally an impedance matching layer 32. A transducer element shown in Figure 8C uses additionally an acoustic lens 33. A transducer element shown in Figure 8D is further provided with an acoustic load member 34 layered on the transducer element 31. In Figure 9, like numerals are used for designating like parts in Figures 8A to 8D and no further explanation is given for simplicity. When the transducer shown in Figure 9 is used in combination with the filter 14 with a variable frequency band, the effective width of the transducer can be changed and the resolution of a tomogram can be improved.

As described above, the present invention can improve a resolution of a tomogram by selecting the width of the transducer according to a depth of the portion under diagnosis of a living body.

## Claims

1. An ultrasonic imaging apparatus comprising: transducer means, including a linear array of transducer elements (31) sequentially disposed along an axis for directing pulses of ultrasonic energy in the form of a beam at a plurality of ultrasonic frequencies into an object and receiving echoes of said pulses to convert said echoes to electrical signals, means (11) for energizing said transducer means, filter means (14) connected to said transducer means for filtering received electrical signals from said transducer means, signal processing means (16) connected to said filter means (14) for processing the frequency components of said electrical signals passed through said filter means (14) to convert them into tomogram signals, and display means (17) connected to said signal processing means (16) for displaying a tomogram according to a tomogram signal from said signal processing means (16), said filter means (14) selectively filtering said electrical signals and passing frequency components of said electrical signals associated with selected frequencies of said ultrasonic energy and not passing other frequency components of said electrical signals, the arrangement of said transducer elements (31) and said filter means (14) cooperating to cause the effective aperature of said transducer means for receiving echoes to vary with the selected frequency components of said electrical signals passed by said filter means (14), characterized in that the thickness of each of said transducer elements (31) varies continuously in a direction orthogonal to said array direction and to said beam so as to increase from a central portion thereof toward both edges.

2. An ultrasonic imaging apparatus according to claim 1, characterized in that said filter means (14) is a band pass filter, the center frequency thereof being variable.

3. An ultrasonic imaging apparatus according to claim 1, characterized in that said filter means (14) is a high pass filter, the cut-off frequency thereof being variable.

## Patentansprüche

1. Ultraschall-Bildwandler oder -Abbildungsgerät, umfassend
eine Wandlereinrichtung mit einem linearen Array von Wandlerelementen (31), die sequentiell längs einer Achse angeordnet sind, um Ultraschallenergie-Impulse in Form eines Strahls auf einer Anzahl von Ultraschallfrequenzen in ein Objekt zu richten und Echos der Impulse zur Umwandlung der Echos in elektrische Signale zu empfangen,
eine Einheit (11) zum Erregen der Wandlereinrichtung,
eine an die Wandlereinrichtung angeschlossene Filtereinheit (14) zum Filtern der von der Wandlereinrichtung empfangenen elektrischen Signale,
eine an die Filtereinheit (14) angeschlossene Signalverarbeitungseinheit (16) zum Verarbeiten der Frequenzkomponenten der durch die Filtereinheit (14) hindurchgelaufenen elektrischen Signale zwecks Umwandlung derselben in Tomogrammsignale und
eine an die Signalverarbeitungseinheit (16) angeschlossene Wiedergabe- oder Anzeigeeinheit (17) zum Wiedergeben bzw. Anzeigen eines Tomogramms nach Maßgabe eines Tomogrammsignals von der Signalverarbeitungseinheit (16),
wobei die Filtereinheit (14) selektiv die elektrischen Signale filtert und Frequenzkomponenten der elektrischen Signale durchläßt, welche gewählten Frequenzen der Ultraschallenergie zugeordnet sind, und andere Frequenzkomponenten der elektrischen Signale nicht durchläßt, wobei in der Anordnung die Wandlerelemente (31) und die Filtereinheit (14) in der Weise zusammenwirken, daß sie die effektive Apertur der Wandlereinrichtung für die empfangenen Echos mit den gewählten Frequenzkomponenten der von der Filtereinheit (14) durchgelassenen elektrischen Signale variieren lassen,
dadurch gekennzeichnet, daß
die Dicke jedes der Wandlerelemente (31) fortlaufend in einer Richtung senkrecht zur Array-Richtung und zum Strahl variiert, so daß sie von einem Mittelbereich davon zu den beiden Rändern hin zunimmt.

2. Ultraschall-Bildwandler nach Anspruch 1, dadurch gekennzeichnet, daß die Filtereinheit (14) ein Bandpaßfilter ist, dessen Mittenfrequenz variabel ist.

3. Ultraschall-Bildwandler nach Anspruch 1, dadurch gekennzeichnet, daß die Filtereinheit (14) ein Hochpaßfilter ist, dessen Grenzfrequenz variabel ist.

**Revendications**

1. Appareil de formation d'images par ultrasons comportant:

un transducteur comprenant un réseau linéaire d'éléments transducteurs (31) disposés séquentiellement lelong d'un axe pour diriger des impulsions d'énergie ultrasonores sous la forme d'un faisceau à plusieurs fréquences ultrasonores dans un objet et pour recevoir des échos desdites impulsions et les convertir en des signaux électriques,

un dispositif (11) pour exciter ledit transducteur,

un filtre (14) connecté audit transducteur pour filtrer des signaux électriques reçus provenant dudit transducteur,

un dispositif de traitement de signaux (16) connecté audit filtre (14) pour traiter les composantes de fréquence desdits signaux électriques qui passent par ledit filtre (14) et pour les convertir en des signaux tomographiques, et

un dispositif de visualisation (17) connecté audit dispositif de traitement de signaux (16) pour visualiser un tomogramme selon un signal tomographique provenant dudit dispositif de traitement de signaux (16),

ledit filtre (14) filtrant sélectivement lesdits signaux électriques et laissant passer des composantes de fréquence desdits signaux électriques associées avec des fréquences sélectionnées de ladite énergie ultrasonore et ne laissant pas passer d'autres composantes de fréquence desdits signaux électriques, la disposition desdits éléments transducteurs (31) et dudit filtre (14) coopérant pour que l'ouverture effective dudit transducteur aux échos reçus varie avec les composantes de fréquence sélectionnées desdits signaux électriques passant par ledit filtre (14), caractérisé en ce que:

l'épaisseur de chacun desdits éléments transducteurs (31) varie continuellement dans une direction perpendiculaire à ladite direction du réseau et dudit faisceau de manière à augmenter depuis une partie centrale de ce réseau vers ses deux extrémités.

2. Appareil de formation d'images par ultrasons selon la revendication 1, caractérisé en ce que ledit filtre (14) est un filtre passe-bande dont la fréquence centrale est variable.

3. Appareil de formation d'images par ultrasons selon la revendication 1, caractérisé en ce que ledit filtre (14) est un filtre passe-haut dont la fréquence de coupure est variable.

# F I G.   1

# F I G.   2

F I G. 3

12'

12

12c1
12b1
12a
12b2
12c2

D4 D5 D6 D2' D3'

ℓ1
Xℓ1
ℓ2
Xℓ2

0 072 498

2

0 072 498

FIG. 4

FIG. 5

FIG. 6

3

# F I G. 7

# FIG. 8A  FIG. 8B  FIG. 8C  FIG. 8D

# F I G. 9